# EUROPÄISCHE PATENTSCHRIFT

(11) **EP 1 127 146 B1**
(45) Veröffentlichungstag und Bekanntmachung des Hinweises auf die Patenterteilung: **21.07.2004**
(21) Anmeldenummer: 99962041.2
(22) Anmeldetag: 27.10.1999
(51) Int. Cl.: C12N 15/82, C12N 15/63, C12N 5/10, A01H 5/00

(54) **EXPRESSIONSKASSETTE ZUR EXPRESSION VON BELIEBIGEN GENEN IN PFLANZENSAMEN**
NOVEL EXPRESSION CASSETTE FOR EXPRESSING GENES IN PLANT SEED
NOUVELLE CASSETTE D'EXPRESSION PERMETTANT D'EXPRIMER DES GENES QUELCONQUES DE GRAINES DE PLANTES

(30) Priorität: 04.11.1998 DE 19852195
(43) Veröffentlichungstag der Anmeldung: 29.08.2001
(73) Patentinhaber: INSTITUT FÜR PFLANZENGENETIK UND KULTURPFLANZENFORSCHUNG, 06466 Gatersleben (DE)
(72) Erfinder: HEIM, Ute, D-06466 Gatersleben (DE); WEBER, Hans, D-06484 Quedlinburg (DE)
(74) Vertreter: Baumbach, Friedrich, Dr.
(86) Internationale Anmeldenummer: PCT/DE1999/003432
(87) Internationale Veröffentlichungsnummer: WO 2000/026388

(56) Entgegenhaltungen:
- WO-A-92/18634
- WO-A-98/53086
- HEIM U ET AL: "Cloning and characterization of full-length cDNA encoding sucrose phosphate synthase from faba bean" GENE: AN INTERNATIONAL JOURNAL ON GENES AND GENOMES,GB,ELSEVIER SCIENCE PUBLISHERS, BARKING, Bd. 178, Nr. 1, 31. Oktober 1996 (1996-10-31), Seiten 201-203, XP004043363 ISSN: 0378-1119
- GRIMES ET AL: "a 62-kD sucrose binding protein is expressed and localized in tissues actively engaged in sucrose transport" PLANT CELL,US,AMERICAN SOCIETY OF PLANT PHYSIOLOGISTS, ROCKVILLE, MD, Nr. 4, 1. Dezember 1992 (1992-12-01), Seiten 1561-1574, XP002079375 ISSN: 1040-4651 in der Anmeldung erwähnt

## Beschreibung

Die vorliegende Erfindung betrifft eine Expressionskassette zur Expression von beliebigen Genen in Pflanzensamen und die die Expressionskassette enthaltenden Plasmide. Die Erfindung schließt die Herstellung transgener Pflanzenzellen, die diese Expressionskassette enthalten, sowie die Verwendung der Plasmide in dieser Expressionskassette zur Herstellung von transgenen Pflanzen mit ein. Anwendungsgebiete der Erfindung sind die Biotechnologie, die Pharmazie und die Pflanzenproduktion.

Seit langem gibt es Methoden, die es ermöglichen, relevante Gene in das Genom höherer Pflanzen einzuschleusen. Ziel dieser Arbeiten ist die Herstellung von Pflanzen mit neuen Eigenschaften, zum Beispiel zur Steigerung der landwirtschaftlichen Produktion, der optimierung der Lebensmittelherstellung und der Produktion bestimmter Pharmazeutika und anderer interessanter Inhaltsstoffe. Eine Voraussetzung für die Expression der übertragenen Gene ist dabei, daß sie über pflanzenspezifische Promotorsequenzen verfügen. So werden dazu bereits sogenannte konstitutive Promotoren wie der Promotor des Nopalinsynthase - Gens /1/, der TR - Doppelpromotor /2/ oder der Promotor des 35S - Transkriptes des Blumenkohl - Mosaikvirus /3/ verwendet. Ein Nachteil dieser Promotoren ist es, daß sie in fast allen Geweben der manipulierten Pflanzen aktiv sind. Dadurch ist eine kontrollierte und gezielte Expression der Fremdgene in den Pflanzen nicht möglich. Es ist besser, Promotoren zu benutzen, die gewebespezifisch und entwicklungsabhängig funktionieren. So wurden Gene mit den dazu gehörigen Promotoren isoliert, die nur in Antheren, Ovarien, Blüten,-Blättern, Laubblättern, Stengeln, Wurzeln oder Samen aktiv sind /4/. Sie unterscheiden sich aber sehr in der Stärke und Spezifität der Expression und sind nur begrenzt einsetzbar. Für die Nutzung der Samen als Ernährungsquelle und zur Produktion von Inhaltsstoffen sind vor allem die samenspezifischen Promotoren von großem Interesse. Durch die langjährige Erforschung der Gene der Samenspeicherproteine stehen schon einige mehr oder weniger spezifische und in der Stärke unterschiedliche Promotoren, wie beispielsweise des Phaseolins /5/ oder des Legumins und USP /6/ zur Verfügung. Da diese Speicherproteine von Genfamilien synthetisiert werden, stehen Fusionen solcher Promotoren mit Fremdgenen in Konkurrenz zu den endogenen zahlreichen Genen der entsprechenden Genfamilie. Deshalb ist es günstiger, Promotoren von unikalen, stark und spezifisch exprimierenden Genen zu benutzen. Für Ko - und Mehrfachtransformationen ist die Verwendung verschiedener regulatorischer Sequenzen angebracht, um die zeitliche Entwickung des Samens besser auszunutzen, parallel gleiche oder verschiedene Genprodukte zu synthetisieren und um Kosuppression zu vermeiden.

Obwohl also bereits mehrere Expressionskassetten zur Expression beliebiger Gene in Pflanzensamen bekannt sind, waren die erreichten Expressionsraten in Pflanzensamen bisher noch nicht optimal, um darauf eine pflanzenbiotechnologische Produktion der gewünschten Stoffe zu begründen.

Die Erfindung hat daher das Ziel, die samenspezifische Expression in transgenen Pflanzen auf eine für eine Stoffproduktion geeignete Basis zu stellen. Ihr liegt die Aufgabe zugrunde, eine Expressionskassette zu konstruieren, mit der eine stabile Expression mit hoher Expressionsrate von Genen der herzustellenden Stoffe in Pflanzensamen erreicht werden kann.

Die Zielstellung der Erfindung wird mit der in Anspruch 1 beschriebenen Expressionskassette erreicht, die Unteransprüche 2-7 sind Vorzugsvarianten.

Die erfindungsgemäße Expressionskassette enthält folgende wesentlichen Bestandteile:
o den Promotor des Gens des Saccharosebindeprotein (SBP)-ähnlichen Samenproteins
o ggf. die DNA-Sequenz eines Signalpeptids, bevorzugt des SBP-Signalpeptids
o ein zu exprimierendes Gen
o 3'-Terminationssequenzen

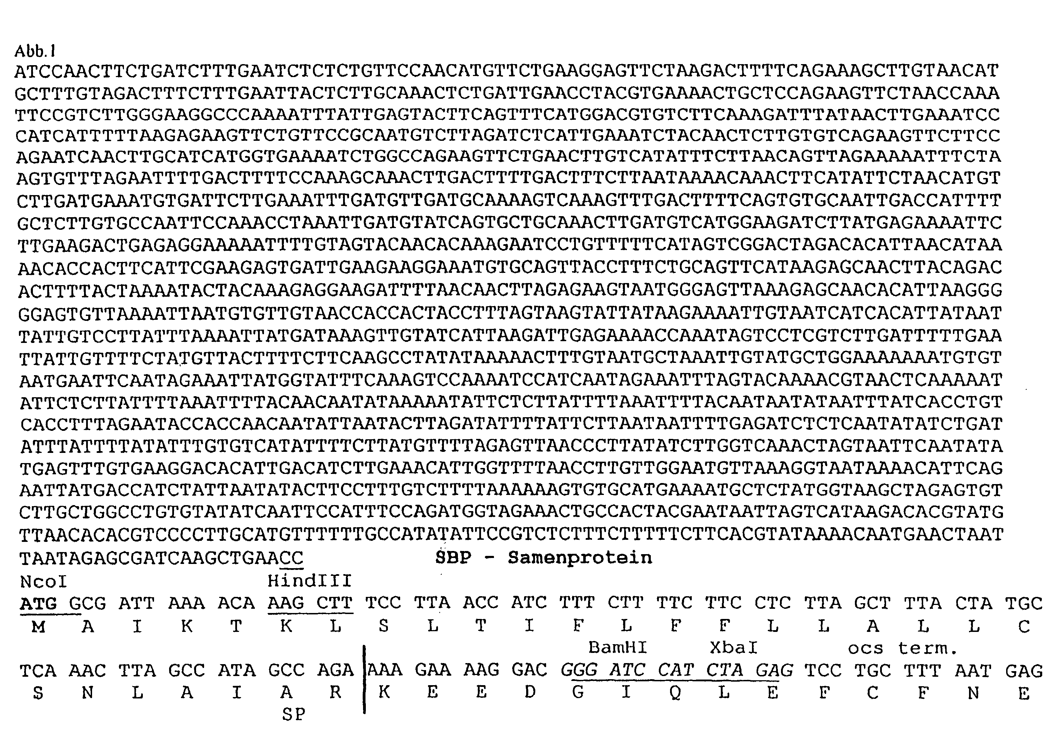

Die Erfindung bezieht sich vor allem auf eine unikal im Genom vorkommende regulatorische DNA - Sequenz, die eine starke Expression eines beliebigen heterologen Gens im wesentlichen in den Keimblättern und im Endosperm von Samen entwicklungsabhängig vermittelt.
Der wichtigste Bestandteil der Kassette ist der SBP-Promotor, dessen Sequenz in der Abb. 1 dargestellt ist. Dieser Promotor hat gegenüber analogen Promotoren auf diesem Gebiet den Vorteil großer Stärke und Samenspezifität. Seine Nutzung für die Expression von Fremdgenen auch ohne die DNA-Sequenz eines Signalpeptids gehört ebenfalls zum Umfang der Erfindung.

Die Expressionskassette enthält neben den transkriptionell regulatorischen Sequenzen ggf. auch ein Signalpeptid, welches den Transport des gewünschten Genproduktes in die Proteinkörper ermöglicht und so einen Abbau der Genprodukte weitgehend verhindert. Die wahlweise Nutzung des authentischen Signalpeptids ermöglicht den Transport des synthetisierten Fremdproteins zu und die Lagerung in den Proteinbodies.

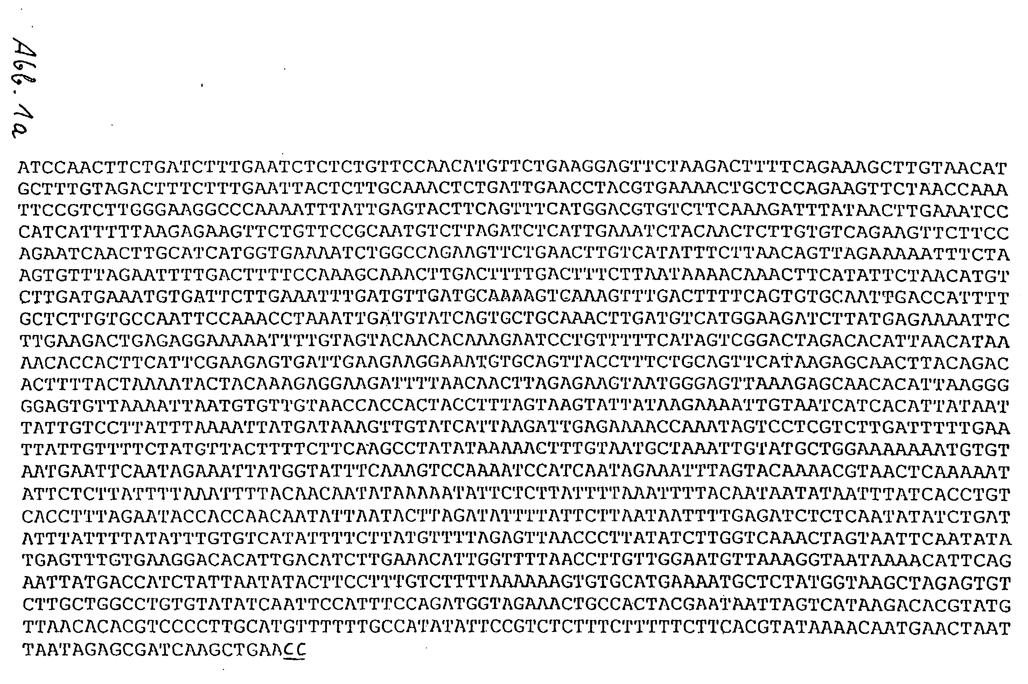

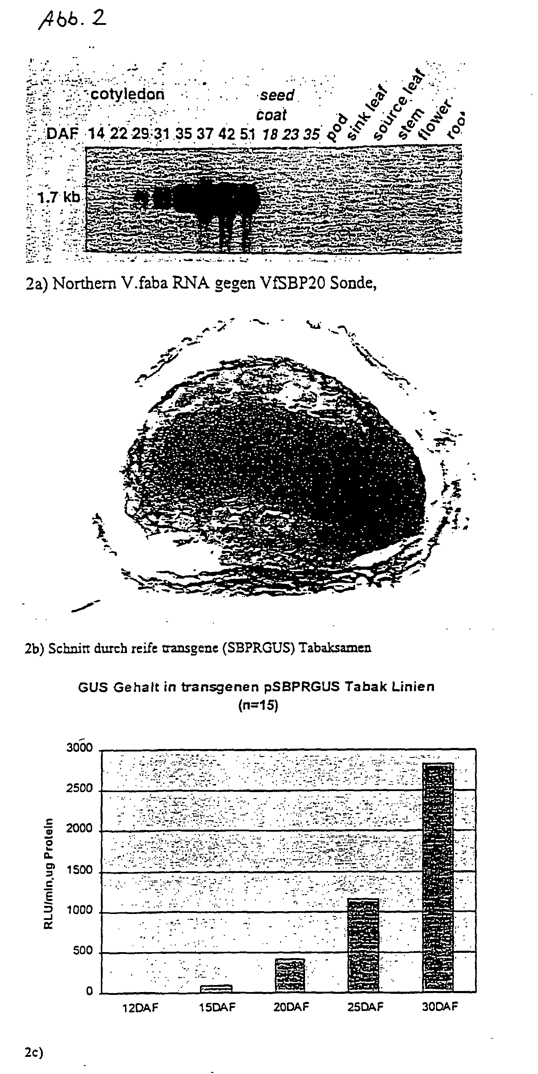

Die zu exprimierenden Gene können entweder als Transkriptions- oder als Translationsfusionen integriert sein, sie können weitgehend variiert werden, beispielsweise können Gene für die Produktion von Enzymen (z.B. Amylase, Xylanase), pharmazeutischer Produkte oder für die Überexpression von Proteinen mit einem hohen Anteil essentieller Aminosäuren (z.B. methioninreiches 2S Globulin der Brasilnuß) oder anderer die Eigenschaften der Samen beeinflussender Proteine eingesetzt werden. Weitere Möglichkeiten liegen in der Reduzierung oder im Ausschalten von Genprodukten durch die Integration von Genen in antisense Orientierung. Durch den Einbau regulatorischer Gene unter Kontrolle dieses samenspezifischen Promotors können auch Stoffwechselprozesse im Samen beeinflußt werden. Die Kassette kann ebenfalls benutzt werden, um das dem Promotor eigene SBP Gen aus der Ackerbohne in anderen Spezies zu exprimieren. Die Nutzung anderer Terminatoren, so zum Beispiel die Terminationssequenz des zu exprimierenden Gens, ist eine weitere Möglichkeit, um die Kassette optimal einzusetzen. Als konkretes Beispiel wurde das Gen der B-Glucuronidase (GUS) genutzt, um die Spezifität des Promotors zu zeigen (Abb. 2b,c).

Die Nukleotidsequenz der Expressionskassette enthält transkriptional regulatorische Bereiche, die eine starke spezifische Expression eines beliebigen Gens in den Samen von Pflanzen gewährleistet. Der Northern (Abb.2a) zeigt die hohe samenspezifische Expression in den verschiedenen Geweben von Vicia faba. Die GUS-Daten in den Abb. 2b und 2c zeigen zum einen in den Schnitten durch reifen Tabaksamen die Verteilung der β-Glucuronidase und zum anderen die entwicklungsabhängige Akkumulation der β-Glucuronidase in den transgenen Tabaksamen.
Unter Schutz gestellt werden auch die Plasmide, welche die Expressionskassette enthalten, bevorzugt die Plasmide pSBPROCS und pPTVSBPRGUS.

Zum Umfang der Erfindung gehört auch die Verwendung der Expressionskassette gemäß Ansprüchen 12-16, die durch Transformation in Bakterienstänne und anschließendem Transfer der entstandenen rekombinanten Klone in vorzugsweise dicotyle Pflanzen erfolgt. Die das gewünschte Genprodukt im Samen exprimierenden Pflanzen werden selektiert und als genetisch stabile Linien gezüchtet. Nach der Ernte werden dann die gewünschten Genprodukte aus den transgenen Samen in an sich bekannter Weise extrahiert.

Diese Erfindung ist auch interessant für Anwendungen, wo das gewünschte Genprodukt unter der Kontrolle verschiedener Promotoren exprimiert wird, um die Expressionsraten in der Summe zu erhöhen, um den Entwicklungszeitraum der Samen besser zu nutzen und um Effekte durch Kosuppression zu vermeiden. Für Ko- und Mehrfachtransformationen mit dem Ziel, verschiedene Genprodukte zu exprimieren, ist diese Expressionskassette ebenfalls geeignet. Für diese Strategien benötigt man eine Vielzahl neuartiger Expressionskassetten, um die richtigen auswählen zu können.

Das gesamte Verfahren zur Veränderung einer Pflanzenzelle wird an einem Beispiel (pSBPOCS) dargestellt.

Die Erfindung soll nachfolgend durch Ausführungsbeispiele näher erläutert werden.

### Methoden

### 1. Klonierungsverfahren

Zur Klonierung wurden die Vektoren pUC18 /7/, pBK-CMV (Stratagene) und pOCS1 (Plant Genetic Systems, Gent, Belgien) und für die Pflanzentransformation die Vektoren BIN19 /8/, sowie nach Deletion des GUS Gens pGPTV-BAR /9/, verwendet.

### 2. Bakterienstämme

Für die Transformation in E. coli wurde der Stamm DH5α /10/ verwendet. Durch Konjugation wurden die Binärplasmide in den Agrobakterienstamm EHA105 /11/ eingeführt.

### 3. Pflanzentransformation

Die Transformation von Nicotiana tabaccum erfolgte durch die Blattscheibchenmethode /12/ und die Transformation von Vicia narbonensis mit Hilfe der von Pickardt 1991 beschriebenen Methode /13/ über Agrobakterien vermittelten Gentransfer.

### 4. Analyse genomischer DNA aus transgenen Pflanzen

Die genomische DNA der transgenen Tabak und V. narbonensis Pflanzen wurde mit Hilfe des DNA - Isolierungskit der Firma Macherey & Nagel isoliert. In einem ersten Schritt wurden die transgenen Linien über PCR mit genspezifischen Primern identifiziert. Die Integration der Fremd-DNA wurde mittels "Southernblot" - Analysen von 20µg DNA nach geeigneter Restriktionsspaltung untersucht.

### 5. β-Glucuronidase - Aktivitätstest (GUS - Assay)

Das Reportergen β-Glucuronidase ist ein bakterielles Enzym, das sowohl quantitativen /14/ als auch histochemischen Aktivitätsbestimmungen zugänglich ist. Gewebeproben wurden in 1mM X-Gluc, 50mM Na-Phosphat (pH 7,0) und 0,1% Tween 20 über Nacht bei 37°C inkubiert. Für Schnitte wurden die Gewebe fixiert, in Paraffin eingebettet und am Mikrotom auf 15 - 30 µm Schnittdicke geschnitten.

### Ausführungsbeispiele

Die Erfindung, die die Herstellung einer neuen samenspezifischen Expressionskassette enthält sowie die sich daraus ableitenden Plasmide und transgenen Pflanzen, wird nachstehend - zum Teil an Hand von den Abbildungen- an einem Ausführungsbeispiel erläutert.

### 1.) Klonierung und Strukturanalyse eines SBP - Samenprotein - Gens aus Vicia faba

Von der Sequenz eines cDNA Klons, der für das Saccharosebindeprotein aus der Sojabohne kodiert /15/, wurden Primer (5'-GAAGACCCTGAGCTCGTAACTTGCAA-ACAC- 3' und 5'-AGTACTCATAGATCTCTGGGTGATGTTGGT-3') abgeleitet. Mittels RT - PCR an mRNA, isoliert aus unreifen Kotyledonen von V. faba, wurde dann die genspezifische Sonde amplifiziert, kloniert und sequenziert. Das PCR - Produkt wurde als dem Saccharosebindeprotein homologes Genfragment identifiziert und diente als Sonde für die Isolierung der vollständigen cDNA aus einer Kodyledonen spezifischen λ Zap Express cDNA Bank aus V. faba L. var. minor. Einer der isolierten Klone (VfSBP20), der auf Nukleotidebene eine Homologie von 68% hat, kodiert für das vollständige SBP homologe Gen aus der Ackerbohne. Es unterscheidet sich aber sowohl in der Expression (Abb.2a) als auch in der Funktion (keine Saccharosebindung) von dem aus der Sojabohne isoliertem Gen.

### 2) Isolierung der regulatorischen Sequenzen mittels PCR

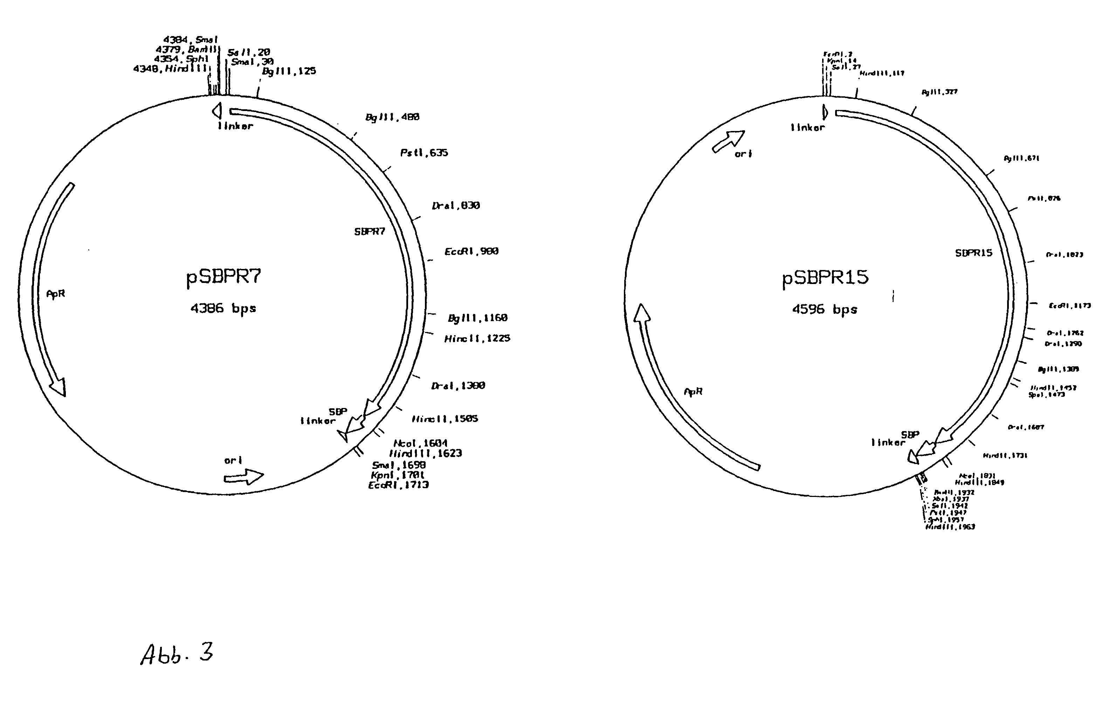

Die regulatorischen Sequenzen wurden mit Hilfe des "Universal GenomeWalker™Kit" der Firma CLONTECH und den genspezifischen Primern PSBP1, Position 159 (5'-AATCCTCA-CACTTCTCCATGCATATCCGTTTGTCC-3'), PSBP2, Position 118 (5'-GCCCTGCAGAT-CGCATTTGTCTTTGCA-3') und PSBP3, Position 85 (5'-CTGGGTCCTTTTCTTTTCTGG- C-3') isoliert. Nach vorheriger Spaltung der genomischen DNA von V.faba mit ScaI (a) bzw. StuI (b) und Ligation der Adaptoren wurde entsprechend der Beschreibung des Kits eine Zweischritt - PCR nach folgenden Parametern durchgeführt: 7 Zyklen a' 94°C, 2s, 72°C, 3 min und 32 Zyklen a' 94°C, 2s, 67°C, 3 min und 4min 67°C. Die PCR - Ansätze wurden 1:50 verdünnt und jeweils 1µl in einer zweiten PCR (5 Zyklen a' 94°C, 2s, 72°C, 3 min und 20 Zyklen a' 94°C, 2s, 67°C und 4min bei 67°C amplifiziert. Im Agarosegel konnten Banden von 1,7kb aus (a) und 1,9kb aus (b) über einen Southernblot verifiziert werden. Diese Banden wurden dann in den pUC18 kloniert und sequenziert. Die Klone SBPR7 und SBPR15 konnten dann durch Sequenzvergleich als die zum Gen VfSBP20 zugehörigen Promotoren identifiziert werden. Sie stellen allelische Varianten des Gens VfSBP20 dar, wobei beide Klone 100% Sequenzidentität im entsprechenden Bereich zum Klon VfSBP20 aufweisen. 5' seitig vom ATG des SBP Gens sind mit dem Klon SBPR7 1539bp und mit dem Klon SBPR15 1750bp isoliert worden. Sie unterscheiden sich durch 23 Basenpaarsubstitutionen und zwei Insertionen. Die Restriktionskarte der Klone pSBPR7 und pSBPR15 sind in Abb. 3, die Sequenz des Klons pSBPR15 ist in Abb. 1 wiedergegeben.

### 3a) Nachweis der samenspezifischen Expression in Tabak

Mit Hilfe des Reportergens der β-Glucuronidase sollte die samenspezifische Expression der isolierten regulatorischen Sequenzen SBPR7 und SBPR15 überprüft werden. Dazu wurde das Binärplasmid pBI101 /14/, welches das promotorlose Glucuronidase Gen hinter einem Polylinker enthält, mit SmaI geschnitten und dephosphoryliert. Aus den Plasmiden pSBPR7 bzw. pSBPR15 wurden mittels einer SalI/NcoI Spaltung die Promotoren isoliert und die Enden geglättet. Die Fragmente wurden dann in den SmaI - Ort des Binärplasmides pBI101 vor das Reportergen kloniert, wobei die Plasmide pBISBPR7GUS und pBISBPR15GUS entstanden sind. Diese Plasmide wurden dann in den Agrobakterienstamm EHA105 transferiert und die chimären SBP-Promotor/Glucuronidase Gen enthaltenen Agrobakterien für die Transformation von Tabak eingesetzt. Die Ergebnisse sind in Figur 2b und 2c abgebildet. Die Analyse der transgenen Tabaksamen zeigt eine starke Blaufärbung und damit eine starke Aktivität der Glucuronidase im Endosperm und in den Keimblättern der Tabaksamen auch entsprechend der Samenentwicklung. In anderen Geweben konnte keine Glucuronidaseaktivität nachgewiesen werden. Auch unterscheiden sich die beiden leicht verschiedenen Nukleotidsequenzen SBPR7 und SBPR15 nicht in ihrem Expressionsverhalten. Diese Daten zeigen, daß die isolierten regulatorischen Sequenzen, die mit dem β-Glucuronidase Gen fusioniert wurden, eine starke und streng samenspezifische Expression im Tabak vermitteln.

### 3b) Nachweis der samenspezifischen Expression in der Erbse

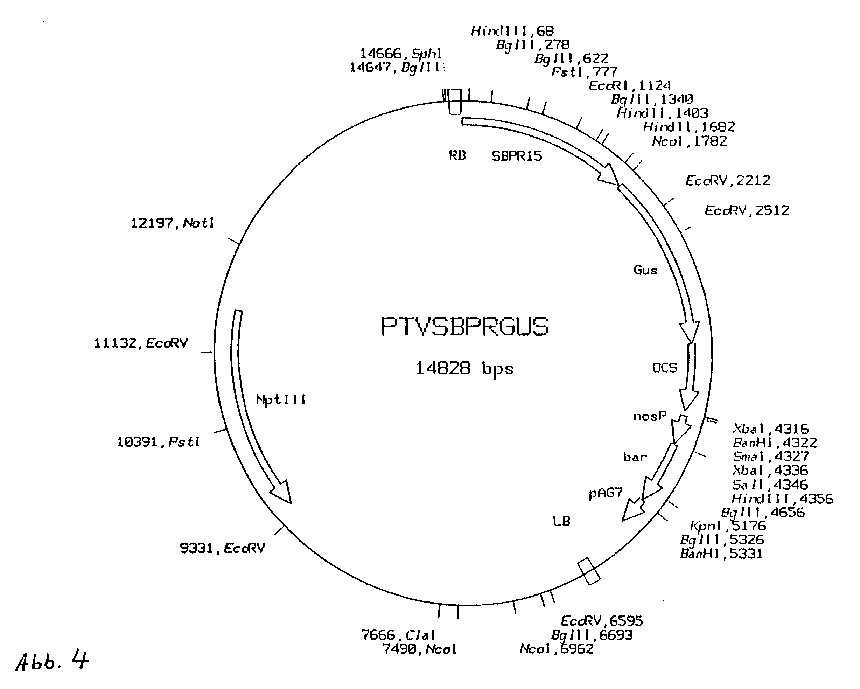

Um zu zeigen, daß auch in den Leguminosen mit einer samenspezifischen Expression zu rechnen ist, wurde das SalI/NcoI Fragment des Plasmids pSBPR15 in das SalI/NcoI geschnittene Plasmid pGUS1 (Plant Genetic Systems, Gent) kloniert. Aus dem resultierenden Plasmid pSBPGUS wurde mit SalI/SmaI die Fusion des SBPR15 Promoters/GUS/ocs-Terminator ausgeschnitten, geglättet und in das Binärplasmid pGPTV-Bar, EcoRI/SmaI geschnitten, ligiert (Abb.4) . pGPTV-Bar /9/ ist ein Phosphinithricin-resistenz vermittelndes Binärplasmid, welches erfolgreich für die Transformation von Erbsen eingesetzt wird. Dieses Plasmid wurde pPTVSBPRGUS (Abb.4) genannt. Die Embryonen der mit diesem Plasmid erzeugten transgenen Erbsenlinien zeigen eine starke Blaufärbung nach histochemischer Analyse.

### 3c) Nachweis der transienten Expression in Embryonen von Vicia faba, Vicia narbonensis, Pisum sativum und Brassica napus

Mit dem Plasmid pSBPGUS wurden isolierte Embryonen von Vicia faba, Vicia narbonensis, Pisum sativum und Brassica napus mittels dem Biolistics PDS-1000/He Particle Delivery System unter folgenden Bedingungen beschossen. Der Coating-Ansatz bestand aus 50µl Gold (Hereaus, 0,6-3µm, 50mg/ml), 10µl Qiagen gereinigte Plasmid-DNA (1µg/µl), 50µl 2,5M CaCl₂ und 10µl 0,1M Spermidine. Bei 1800 Psi und einem Vakuum von 27 inch Hg wurden dann die auf einer Agarplatte liegenden Embryonen beschossen, die anschließend in MS-2% Sucrose Flüssigmedium für 2 Tage kultiviert wurden. Dann erfolgte die Reaktion mit X-Gluc (1mM) in 50mM Na-Phosphat (pH 7,0) und 0,1% Tween 20 über Nacht bei 37°C. Im Gegensatz zur Negativkontrolle (promoterloses pGUS1) konnten viele blaue Punkte bei den obengenannten Embryonen registriert werden, die zeigen, daß der SBP-Promoter in den Samen funktioniert.

### 4.) Herstellung der Expressionskassette zur Überexpression von heterologen Genen in Samen

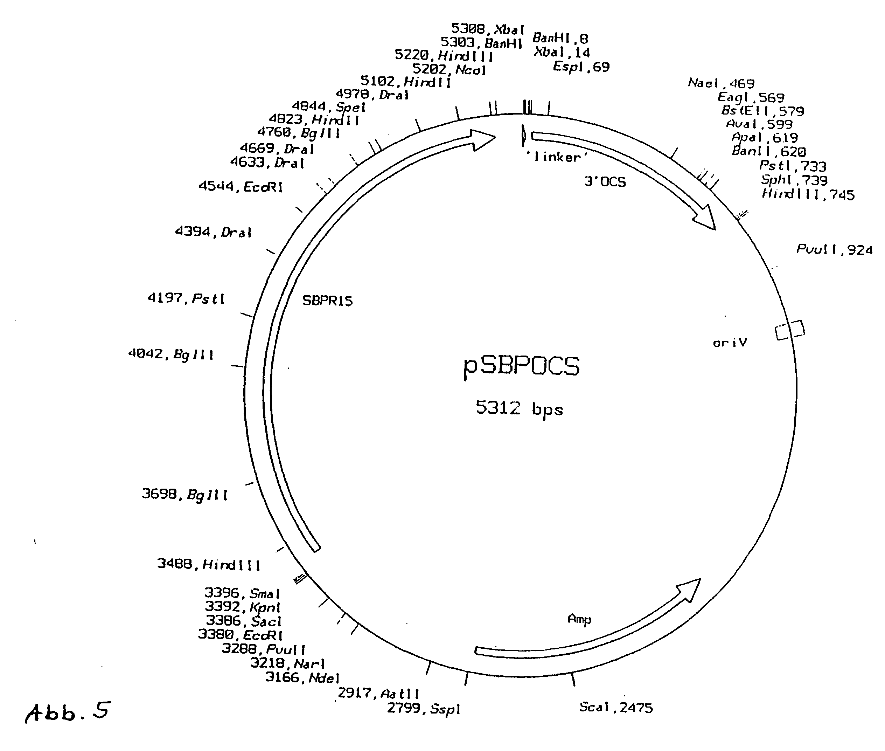

Um die regulatorischen Sequenzen für die Überexpression von Fremdgenen verfügbar zu machen, wurde das SalI Fragment des längeren Klons SBPR15 isoliert und geglättet und in den SmaI Ort des Plasmides pOCS1 (Plant Genetic Systems, Gent, Belgien) kloniert. Diese Kassette enthält somit die Promotorregion, die vollständige 5' untranslatierte Region, das vollständige Signalpeptid, die ersten fünf Triplets des reifen Proteins (Abb. 1) und den 3' untranslatierten Bereich mit den Polyadenylierungssignalen des Octopin Synthase Gens (Fig.5). Für Transkriptionsfusionen mit Fremdgenen kann der NcoI-Ort, für Translationsfusionen der BamHI -Ort genutzt werden. Nach erfolgter Insertion des Fremdgens wird die den Promoter, regulatorische Sequenzen ,das Fremdgen und die 3'-Terminationssequenzen enthaltene Sequenz mit Restriktionsenzymen ausgeschnitten und in einen Binärvektor mit der für die Pflanzentransformation geeigneten Herbizidresistenz kloniert.

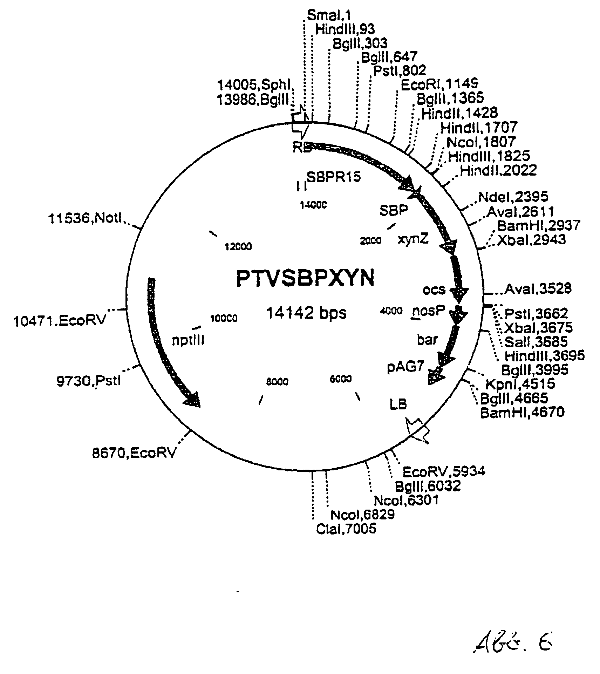

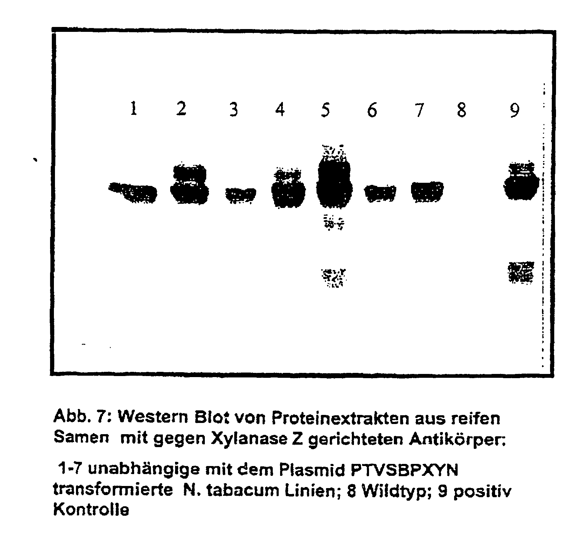

Als Beispiel dafür wurde das BamHI-Fragment des Gens der XylanaseZ von Clostridium thermocellum in den BamHI-Ort des Plasmids pSBPOCS als Translationsfusion kloniert. Aus dem resultierenden Plasmid pSBPRXYNZ (Abb. 6) wurde das geglättete Asp718/SphI Fragment mit dem mit den Enzymen EcoRI/SmaI geschnitten und geglätteten Binärvektor pGPTV-Bar ligiert. Nach Transformation in den Agrobakterienstamm EHA105 wurde N. Tabacum transformiert. In den reifen transgenen Samen konnte im Western Blot die starke Expression der Xylanase Z gezeigt werden (Abb. 7).

Literatur:
1. Herrera-Estrella, L., Depicker, A., Van Montagu, M. and Schell,J. (1983) Nature, 303, No.5914, 209-213.
2. Velten,J., Velten, L., Hani,R. and Schull,J. (1984) EMBO J. 3, 2723-2730.
3. Koziel,M.G., Adams,T.L., Hazlet,M.A., Damm,D., Miller,J., Dahlbeck,D., Jayne,S. and Staskawicz, B.J. (1984) Journ. of Molec. and Appl. Genet. 2, 549-562.
4. Goldberg,R.B. (1986) Phil. Trans. R. Soc. Lond. B314, 343-353.
5. Hall, T. C. et al (1996) US Patent 5,504,200
6. Conrad,U. et al. (19--) deutsches Patent DE 196 04 588.6
7. Yanisch-Perron,C., Vieira, J. and Messing,J. (1985) Gene, 33, 103-119.
8. Bevan,M. (1984) Nucl. Acids Res. 12, 8711-8720.
9. Becker,D., Kemper,E., Schell,J. and Masterson,R. (1992) Plant Mol. Biol. 20, 1195-1197.
10. Hanahan,D. (1983) J. Mol. Biol. 166, 557-580.
11. Hood,E.E., Gelvin,S.B., Melchers,L.S. and Hoekema,A. (1993) Transgenic. Res. 2, 208-218.
12. Bäumlein,H., Boerjan,W., Nagy, I. , Bassüner, R., Van Montagu,M., Inze,D. and Wobus,U. (1991) Mol Gen. Genet, 225, 459-467.
13. Pickardt,T., Meixner,M., Schade, V. and Schieder, O. (1991) Plant Cell Report, 9, 535-538.
14. Jefferson,R.A. (1987) Plant Molec. Biol. Rep. 5, 387-405.
15. Grimes,H.D., Overvoorde, P.J., Ripp,K., Franceschi, V.R. and Hitz,W.D. (1992) The Plant Cell, 4, 1561-1574.

## Patentansprüche

1. Promotor zur Expression beliebiger Gene in Pflanzensamen **gekennzeichnet durch** die Sequenz der Abb. 1a, die damit Gegenstand des Anspruchs wird.

2. Promotor nach Anspruch 1, **dadurch gekennzeichnet, daß** er die Expression in den Keimblättern und im Endosperm von Samen entwicklungsabhängig vermittelt.

3. Expressionskassette zur Expression von beliebigen Genen in Pflanzensamen enthaltend
• einen Promotor gemäß Anspruch 1 oder 2,
• ein zu exprimierendes Gen
• 3'-Terminationssequenzen.

4. Expressionkassette nach Anspruch 3, **dadurch gekennzeichnet, daß** sie zusätzlich die DNA-Sequenz eines Signalpeptids, bevorzugt des Signalpeptids des Saccharosebindeprotein (SBP)ähnlichen -Samenproteins, enthält.

5. Expressionkassette nach Anspruch 3, **dadurch gekennzeichnet, daß** der mit einer transkriptional regulatorischen Sequenz für eine starke samenspezifische Genexpression versehenen DNA - Region eine weitere DNA Sequenz nachgeschaltet ist, die die Information für die Bildung und mengenmäßige Verteilung endogener Produkte oder die Expression heterologer Produkte in Kulturpflanzen enthält.

6. Expressionkassette nach Anspruch 3 bis 5, **dadurch gekennzeichnet, daß** beliebige Fremdgene entweder als Transkriptions- oder als Translationsfusionen integriert sind.

7. Expressionkassette nach Anspruch 3 bis 6, **dadurch gekennzeichnet, daß** die DNA-Sequenz des Signalpeptids des Saccharosebindeprotein(SBP) ähnlichen Samenproteins verwendet wird.

8. Expressionskassette nach Anspruch 3 bis 7, **dadurch gekennzeichnet, daß** als das zu exprimierende Gen das Gen kodierend für das Saccharosebindeprotein(SBP) ähnliche-Samenprotein eingesetzt wird.

9. Expressionskassette nach Anspruch 3 bis 8, **dadurch gekennzeichnet, daß** sie auch für Ko- und Mehrfachtransformationen eingesetzt wird.

10. Plasmide enthaltend eine Expressionskassette gemäß den Ansprüchen 3 bis 8.

11. Plasmid (pSBPOCS) gemäß Abb. 5 nach Anspruch 10, hergestellt durch Klonierung des geglätteten SaIl-Fragments des Plasmids pSPBR15 (gemäß Abb. 3) in den SmaI Ort des Plasmids pOCS1, und enthaltend die Promoterregion, die vollständige 5' untranslatierte Region und das vollständige Signalpeptid des Saccharosebindeprotein (SBP) ähnlichen Samenproteingens, und die ersten fünf Triplets des reifen Proteins (Abb. 1), sowie den 3' untranslatierten Bereich mit den Polyadenylierungssignalen des Octopin Synthase Gens.

12. Plasmid (pPTVSBPRGUS) gemäß Abb. 4 nach Anspruch 10, hergestellt durch Klonierung des SaIl/NcoI-Fragments des Plasmids pSBPR15 (gemäß Abb. 3) in des SaIl/NcoI geschnittene Plasmid pGUS1, nachfolgendem Ausschneiden der Fusion des SBPR15 Promoters/GUS/ocs-Terminators aus dem resultierenden Plasmid pSBPGUS mit SaIl/SmaI, Glättung und Ligierung des entstandenen DNA Fragmentes in das EcoRI/SmaI geschnittene und geglättete Binärplasmid pGPTV-Bar.

13. Verwendung des Plasmids gemäß Anspruch 11 zur samenspezifischen Genexpression in einer Pflanzenzelle.

14. Verwendung einer Expressionskassette gemäß den Ansprüchen 3 bis 9 zur Expression homologer und heterologer Gene in Samen transformierter Pflanzen.

15. Verwendung einer Expressionskassette gemäß den Ansprüchen 3 bis 9 zur Expression von Genen, die die Lagereigenschaften oder die Keimfähigkeit von Samen verändern.

16. Verwendung der Plasmide gemäß Abb. 4 und Abb. 5 oder abgeleitete Plasmide davon zur Transformation von Kulturpflanzen.

17. Verwendung der Plasmide gemäß Abb.4 und Abb. 5 oder davon abgeleitete Plasmide zur Regulation endogener Prozesse oder zur Herstellung heterogener Produkte in Kulturpflanzen.

18. Verwendung einer Expressionskassette gemäß den Ansprüchen 3 bis 9, **dadurch gekennzeichnet, daß** die transformierten Pflanzen, die im Samen veränderte oder neue Genprodukte exprimieren, selektiert, genetisch stabile Linien gezüchtet und die Genprodukte aus den Samen der transgenen Pflanzen extrahiert werden.

19. Pflanzenzelle, enthaltend ein Plasmid gemäß Anspruch 10 bis 12.

20. Pflanzenzelle, hergestellt nach dem Verfahren des Anspruchs 13.

21. Pflanze oder pflanzliche Gewebe, regeneriert aus einer Planzenzelle gemäß der Ansprüche 14 oder 15.

22. Pflanze gemäß Anspruch 21, **dadurch gekennzeichnet, daß** sie eine Kulturpflanze ist.

## Revendications

1. Promoteur pour assurer l'expression de nouveaux gènes quelle qu'en soit la nature dans des semences par la séquence de l'illustration 1a qui devient ainsi l'objet de la revendication.

2. Promoteur selon la revendication 1 **se caractérisant par le fait qu'**il transmet l'expression dans les cotylédons et dans l'endosperme de semences en fonction du développement.

3. Cassette d'expression pour assurer l'expression de nouveaux gènes quelle qu'en soit la nature
dans des semences végétales contenant
• un promoteur selon la revendication 1 ou 2,
• un gène à exprimer
• des séquences de terminaison 3'

4. Cassette d'expression selon la revendication 3, **se caractérisant par le fait qu'**elle contient en plus la séquence ADN d'un peptide de signal, de préférence du peptide de signal de la protéine de la semence, similaire à la protéine de liaison du saccharose (SBP).

5. Cassette d'expression selon la revendication 3, **se caractérisant par le fait que** la région ADN munie d'une séquence régulatrice de transcription pour assurer une forte expression du gène spécifique de la semence est suivie d'une autre séquence ADN qui contient l'information nécessaire pour la formation et la répartition quantitative de produits endogènes ou l'expression de produits hétérologues dans des plantes cultivées.

6. Cassette d'expression selon les revendications 3 à 5, **se caractérisant par le fait que** n'importe quels gènes externes sont intégrés soit en tant que fusions de transcription soit en tant que fusions de traduction.

7. Cassette d'expression selon les revendications 3 à 6, **se caractérisant par le fait que** la séquence ADN du peptide de signal de la protéine de la semence, similaire à la protéine de liaison du saccharose (SBP), est utilisée.

8. Cassette d'expression selon les revendications 3 à 7, **se caractérisant par le fait que** le gène codant la protéine de la semence similaire à la protéine de liaison du saccharose (SBP) est utilisé comme gène à exprimer.

9. Cassette d'expression selon les revendications 3 à 8, **se caractérisant par le fait qu'**elle est aussi employée pour les cotransformations et les transformations multiples.

10. Plasmide contenant une cassette d'expression selon les revendications 3 à 8.

11. Plasmide (pSBPOCS) selon l'illustration 5 selon la revendication 10,
fabriqué par clonage du fragment du site SaIl lissé du plasmide pSBPR15 (selon Illustration 3) dans le site SmaI du plasmide pOCS1, et contenant la région du promoteur, la région non translatée 5' intégrale et le peptide de signal intégral, et les cinq premiers triplets de la protéine mature (III. 1) ainsi que la zone non translatée 3'avec les signaux de polyadénylation du gène de l'octopine synthase.

12. Plasmide (pPTVSBPRGUS) selon l'ill. 4 selon la revendication 10,
fabriqué par clonage du fragment SaIl/NcoI du plasmide pSBPR15 (selon III. 3) dans le plasmide pGUS1 coupé dans le site SaIl/NcoI, suivi de la découpe de la fusion du promoteur/GUS/ocs-terminateur SBPR15 dans le plasmide résultant pSBPGUS avec le site SAIl/SmaI, du lissage et de la ligation du fragment DNA en résultant dans le plasmide binaire pGPTV-Bar coupé et lissé EcoRI/SmaI.

13. Emploi du plasmide selon la revendication 11 pour assurer l'expression du gène spécifique de la semence dans une cellule végétale.

14. Emploi d'une cassette d'expression selon les revendications 3 à 9 pour l'expression de gènes homologues et hétérologues dans les plantes transformées en semence.

15. Emploi d'une cassette d'expression selon les revendications 3 à 9 pour l'expression de gènes qui modifient les propriétés d'entreposage ou le pouvoir germinatif de semences.

16. Emploi des plasmides selon l'ill. 4 et l'ill. 5 ou de plasmides en dérivant pour assurer la transformation de plantes cultivées.

17. Emploi des plasmides selon l'ill. 4 et l'ill. 5 ou de plasmides en dérivant pour assurer la régulation de processus endogènes ou pour la fabrication de produits hétérogènes dans des plantes cultivées.

18. Emploi d'une cassette d'expression selon les revendications 3 à 9, **se caractérisant par le fait que** les plantes transformées qui expriment des produits génétiques nouveaux ou modifiés dans les semences, sont sélectionnées, que des lignes génétiques stables sont cultivées et que les produits génétiques sont extraits des semences de plantes transgéniques.

19. Cellule végétale, contenant un plasmide selon les revendications 10 à 12.

20. Cellule végétale, fabriquée selon le procédé de la revendication 13.

21. Plante ou tissu végétal, régénéré à partir d'une cellule végétale selon les revendications 14 ou 15.

22. Plante selon la revendication 21, **se caractérisant par le fait qu'**elle est une plante cultivée.

## Claims

1. Promoter for the expression of arbitrary genes in plant seeds, wherein there exists the sequence of Fig. 1 a, which thus becomes the object of the claim.

2. Promoter according to Claim 1, wherein it mediates the expression in the cotyleda and in the endosperm of seeds as a function of development.

3. Expression cassette for the expression of arbitrary genes in the plant seed, comprising
• a promoter according to Claim 1 or 2,
• a gene to be expressed
• 3' termination sequences.

4. Expression cassette according to Claim 3, wherein it additionally contains the DNA sequence of a signal peptide, preferably the signal peptide of the seed protein similar to the sucrose binding protein (SBP).

5. Expression cassette according to Claim 3, wherein the DNA region provided with a transcriptionally regulatory sequence for a strong, seed-specific gene expression is followed by a further DNA sequence containing the information for the formation and quantitative distribution of endogenous products or the expression of heterologous products in cultivated plants.

6. Expression cassette according to Claim 3 to 5, wherein arbitrary outside genes are integrated either as transcription or as translation fusions.

7. Expression cassette according to Claim 3 to 6, wherein the DNA sequence of the signal peptide of the seed protein similar to the sucrose binding protein (SBP) is used.

8. Expression cassette according to Claim 3 to 7, wherein the gene coding for the seed protein similar to the sucrose binding protein (SBP) is used as the gene to be expressed.

9. Expression cassette according to Claim 3 to 8, wherein it is also used for co- and multiple transformations.

10. Plasmid containing an expression cassette according to the Claims 3 to 8.

11. Plasmid (pSBPOCS) according to Fig. 5 according to Claim 10,
produced by cloning the smoothed SaIl fragment of the plasmid pSBPR15 (according to Fig. 3) into the SmaI location of the plasmid pOCS1 and containing the promoter region, the complete 5' untranslated region and the complete signal peptide, and the first five triplets of the mature protein (Fig. 1) as well as the 3' untranslated area with the polyadenylation signals of the octopine synthase gene.

12. Plasmid (pPTVSBPRGUS) according to Fig. 4 according to Claim 10,
produced by cloning the SaIl/NcoI fragment of the plasmid pSBPR15 (according to Fig. 3) into the SaIl/NcoI cut plasmid pGUS1, subsequent excision of the fusion of the SBPR15 promoter/GUS/ocs terminator from the resultant plasmid pSBPGUS with SaIl/SmaI, smoothing and ligation of the resultant DNA fragment into the EcoRI/SmaI cut and smoothed binary plasmid pGPTV-Bar.

13. Use of the plasmid according to Claim 11 for seed-specific gene expression in a plant cell.

14. Use of an expression cassette according to the Claims 3 to 9 for expression of homologous and heterologous genes in seeds of transformed plants.

15. Use of an expression cassette according to the Claims 3 to 9 for expression of genes changing the storage properties or the germinability of seeds.

16. Use of the plasmids according to Fig. 4 and Fig. 5 or derived plasmids thereof for transformation of cultivated plants.

17. Use of the plasmids according to Fig. 4 and Fig. 5 or plasmids derived therefrom for regulation of endogenous processes or for production of heterogenous products in cultivated plants.

18. Use of an expression cassette according to the Claims 3 to 9, wherein the transformed plants expressing modified or new gene products in the seed are selected, genetically stable lines are bred and the gene products are extracted from the seeds of the transgenic plants.

19. Plant cell containing a plasmid according to Claims 10 to 12.

20. Plant cell produced according to the method of Claim 13.

21. Plant or plant tissue regenerated from a plant cell according to Claim 14 or 15.

22. Plant according to Claim 21, wherein it is a cultivated plant.


### SEQUENZPROTOKOLL

<110> Inst. f. Pflanzengen. u. Kulturpflanzenforschung
<120> Neue Expressionskassette zur Expression von beliebigen Genen in Pflanzensamen
<130> PCT/DE99/03432
<140> PCT/DE99/03432
<141> 1999-10-27
<150> DE 198 52 195.2
<151> 1998-11-04
<160> 7
<170> Patentin Ver. 2.1
<210> 1
<211> 1783
<212> DNA
<213> vicia faba
<220>
<221> promoter
<222> (1)..(1783)
<400> 1
   atccaacttc tgatctttga atctctctgt tccaacatgt tctgaaggag ttctaagact 60
   tttcagaaag cttgtaacat gctttgtaga ctttctttga attactcttg caaactctga 120
   ttgaacctac gtgaaaactg ctccagaagt tctaaccaaa ttccgtcttg ggaaggccca 180
   aaatttattg agtacttcag tttcatggac gtgtcttcaa agatttataa cttgaaatcc 240
   catcattttt aagagaagtt ctgttccgca atgtcttaga tctcattgaa atctacaact 300
   cttgtgtcag aagttcttcc agaatcaact tgcatcatgg tgaaaatctg gccagaagtt 360
   ctgaacttgt catatttctt aacagttaga aaaatttcta agtgtttaga attttgactt 420
   ttccaaagca aacttgactt ttgactttct taataaaaca aacttcatat tctaacatgt 480
   cttgatgaaa tgtgattctt gaaatttgat gttgatgcaa aagtcaaagt ttgacttttc 540
   agtgtgcaat tgaccatttt gctcttgtgc caattccaaa cctaaattga tgtatcagtg 600
   ctgcaaactt gatgtcatgg aagatcttat gagaaaattc ttgaagactg agaggaaaaa 660
   ttttgtagta caacacaaag aatcctgttt ttcatagtcg gactagacac attaacataa 720
   aacaccactt cattcgaaga gtgattgaag aaggaaatgt gcagttacct ttctgcagtt 780
   cataagagca acttacagac acttttacta aaatactaca aagaggaaga ttttaacaac 840
   ttagagaagt aatgggagtt aaagagcaac acattaaggg ggagtgttaa aattaatgtg 900
   ttgtaaccac cactaccttt agtaagtatt ataagaaaat tgtaatcatc acattataat 960
   tattgtcctt atttaaaatt atgataaagt tgtatcatta agattgagaa aaccaaatag 1020
   tcctcgtctt gatttttgaa ttattgtttt ctatgttact tttcttcaag cctatataaa 1080
   aactttgtaa tgctaaattg tatgctggaa aaaaatgtgt aatgaattca atagaaatta 1140
   tggtatttca aagtccaaaa tccatcaata gaaatttagt acaaaacgta actcaaaaat 1200
   attctcttat tttaaatttt acaacaatat aaaaatattc tcttatttta aattttacaa 1260
   taatataatt tatcacctgt cacctttaga ataccaccaa caatattaat acttagatat 1320
   tttattctta ataattttga gatctctcaa tatatctgat atttatttta tatttgtgtc 1380
   atattttctt atgttttaga gttaaccctt atatcttggt caaactagta attcaatata 1440
   tgagtttgtg aaggacacat tgacatcttg aaacattggt tttaaccttg ttggaatgtt 1500
   aaaggtaata aaacattcag aattatgacc atctattaat atacttcctt tgtcttttaa 1560
   aaaagtgtgc atgaaaatgc tctatggtaa gctagagtgt cttgctggcc tgtgtatatc 1620
   aattccattt ccagatggta gaaactgcca ctacgaataa ttagtcataa gacacgtatg 1680
   ttaacacacg tccccttgca tgttttttgc catatattcc gtctctttct ttttcttcac 1740
   gtataaaaca atgaactaat taatagagcg atcaagctga acc                   1783
<210> 2
<211> 1909
<212> DNA
<213> Vicia faba
<220>
<221> promoter
<222> (1)..(1783)
<220>
<221> CDS
<222> (1784)..(1867)
<223> Signalpeptid
<220>
<221> CDS
<222> (1868)..(1879)
<223> 4 Aminosäuren des reifen SBP-Proteins
<220>
<221> CDS
<222> (1880)..(1894)
<223> BamHI-Xbal Linker
<220>
<221> CDS
<222> (1895)..(1909)
<223> ersten 15 Basenpaare des Terminators der Octopinsynthase
<220>
<221> CDS
<222> (1895)..(1909)
<400> 2
   atccaacttc tgatctttga atctctctgt tccaacatgt tctgaaggag ttctaagact 60
   tttcagaaag cttgtaacat gctttgtaga ctttctttga attactcttg caaactctga 120
   ttgaacctac gtgaaaactg ctccagaagt tctaaccaaa ttccgtcttg ggaaggccca 180
   aaatttattg agtacttcag tttcatggac gtgtcttcaa agatttataa cttgaaatcc 240
   catcattttt aagagaagtt ctgttccgca atgtcttaga tctcattgaa atctacaact 300
   cttgtgtcag aagttcttcc agaatcaact tgcatcatgg tgaaaatctg gccagaagtt 360
   ctgaacttgt catatttctt aacagttaga aaaatttcta agtgtttaga attttgactt 420
   ttccaaagca aacttgactt ttgactttct taataaaaca aacttcatat tctaacatgt 480
   cttgatgaaa tgtgattctt gaaatttgat gttgatgcaa aagtcaaagt ttgacttttc 540
   agtgtgcaat tgaccatttt gctcttgtgc caattccaaa cctaaattga tgtatcagtg 600
   ctgcaaactt gatgtcatgg aagatcttat gagaaaattc ttgaagactg agaggaaaaa 660
   ttttgtagta caacacaaag aatcctgttt ttcatagtcg gactagacac attaacataa 720
   aacaccactt cattcgaaga gtgattgaag aaggaaatgt gcagttacct ttctgcagtt 780
   cataagagca acttacagac acttttacta aaatactaca aagaggaaga ttttaacaac 840
   ttagagaagt aatgggagtt aaagagcaac acattaaggg ggagtgttaa aattaatgtg 900
   ttgtaaccac cactaccttt agtaagtatt ataagaaaat tgtaatcatc acattataat 960
   tattgtcctt atttaaaatt atgataaagt tgtatcatta agattgagaa aaccaaatag 1020
   tcctcgtctt gatttttgaa ttattgtttt ctatgttact tttcttcaag cctatataaa 1080
   aactttgtaa tgctaaattg tatgctggaa aaaaatgtgt aatgaattca atagaaatta 1140
   tggtatttca aagtccaaaa tccatcaata gaaatttagt acaaaacgta actcaaaaat 1200
   attctcttat tttaaatttt acaacaatat aaaaatattc tcttatttta aattttacaa 1260
   taatataatt tatcacctgt cacctttaga ataccaccaa caatattaat acttagatat 1320
   tttattctta ataattttga gatctctcaa tatatctgat atttatttta tatttgtgtc 1380
   atattttctt atgttttaga gttaaccctt atatcttggt caaactagta attcaatata 1440
   tgagtttgtg aaggacacat tgacatcttg aaacattggt tttaaccttg ttggaatgtt 1500
   aaaggtaata aaacattcag aattatgacc atctattaat atacttcctt tgtcttttaa 1560
   aaaagtgtgc atgaaaatgc tctatggtaa gctagagtgt cttgctggcc tgtgtatatc 1620
   aattccattt ccagatggta gaaactgcca ctacgaataa ttagtcataa gacacgtatg 1680
   ttaacacacg tccccttgca tgttttttgc catatattcc gtctctttct ttttcttcac 1740
   gtataaaaca atgaactaat taatagagcg atcaagctga acc atg gcg att aaa   1795
                                                   Met Ala Ile Lys
                                                   1
   aca aag ctt tcc tta acc atc ttt ctt ttc ttc ctc tta gct tta cta   1843
   Thr Lys Leu Ser Leu Thr Ile Phe Leu Phe Phe Leu Leu Ala Leu Leu
   5                   10                  15                  20
   tgc tca aac tta gcc ata gcc aga aaa gaa aag gac ggg atc cat cta   1891
   Cys Ser Asn Leu Ala Ile Ala Arg Lys Glu Lys Asp Gly Ile His Leu
                   25                  30                  35
   gag tcc tgc ttt aat gag                                           1909
   Glu Ser Cys Phe Asn Glu
               40
<210> 3
<211> 28
<212> PRT
<213> Vicia faba
<400> 3
   Met Ala Ile Lys Thr Lys Leu Ser Leu Thr Ile Phe Leu Phe Phe Leu
   1               5                   10                  15
   Leu Ala Leu Leu Cys Ser Asn Leu Ala Ile Ala Arg
               20                  25
<210> 4
<211> 4
<212> PRT
<213> Vicia faba
<400> 4
   Lys Glu Lys Asp
   1
<210> 5
<211> 5
<212> PRT
<213> Vicia faba
<400> 5
   Gly Ile His Leu Glu
   1 5
<210> 6
<211> 5
<212> PRT
<213> Vicia faba
<400> 6
   Ser Cys Phe Asn Glu
   1 5
<210> 7
<211> 5
<212> PRT
<213> Vicia faba
<400> 7
   Ser Cys Phe Asn Glu
   1 5